# EUROPEAN PATENT SPECIFICATION

(11) **EP 3 490 424 B1**
(45) Date of publication and mention of the grant of the patent: **21.05.2025**
(21) Application number: 17746310.6
(22) Date of filing: 20.07.2017
(51) Int. Cl.: A61B 1/00, A61B 1/005

(54) **BRAKE MECHANISM OF A STEERABLE CATHETER**
BREMSMECHANISMUS EINES LENKBAREN KATHETERS
MÉCANISME DE FREINAGE D'UN CATHÉTER ORIENTABLE

(30) Priority: 28.07.2016 US 201662367918 P
(43) Date of publication of application: 05.06.2019
(73) Proprietor: Cook Medical Technologies LLC, Bloomington, IN 47404 (US)
(72) Inventor: SIMMONS, Victor, Mount Airy, NC 27030 (US); DILLON, Travis, E., Winston-Salem, NC 27104 (US)
(74) Representative: Mathys & Squire
(86) International application number: PCT/US2017/043060
(87) International publication number: WO 2018/022418

(56) References cited:
- JP-A- 2005 160 790
- US-A1- 2002 019 591
- US-A1- 2002 099 266

## Description

### TECHNICAL FIELD

Embodiments disclosed herein generally relate to steerable catheters, including endoscopes. More particularly embodiments disclosed herein relate to a structures and methods for a brake that will lock into place actuation elements of a steerable catheter.

### BACKGROUND

Deflecting catheters, also referred to as steerable catheters are used in a variety of medical and non-medical procedures. In diagnostic and therapeutic medical procedures, a steerable catheter provides an operator (e.g., physician) with the ability to articulate the distal tip of the catheter in order to travel through constrained and/or tortuous anatomy, and/or to direct the distal catheter tip in a particular direction. Similar mechanisms are used in medical and nonmedical endoscopes to steer them to a target site and to orient a device portion (e.g., including a camera or other visualization means) in a desired direction.

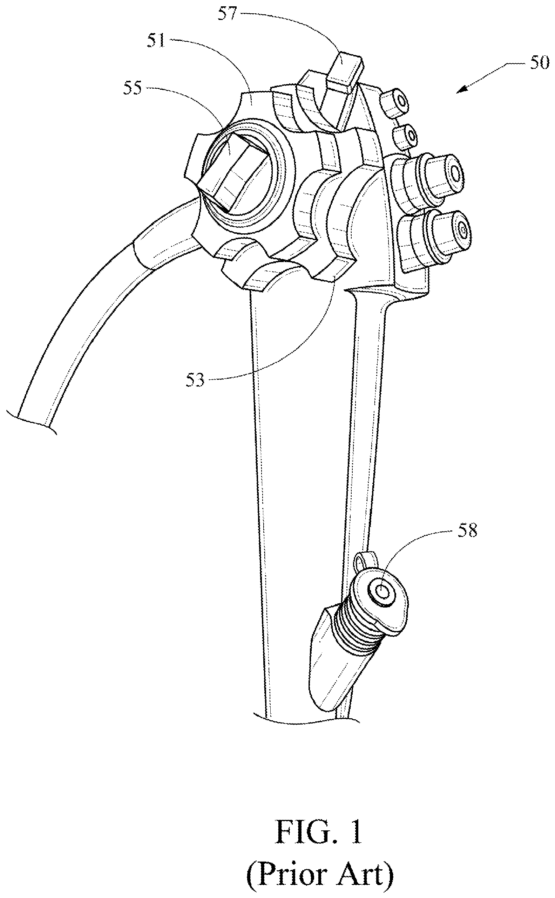

In a typical design, control wires are manipulably attached at a proximal end of the device, and also attached at or near a distal end of the device. Such a configuration operates by manipulating one or more of the control wires to increase and/or decrease a generally longitudinal force on the distal device end that will deflect it in a desired direction. As described with reference to an existing steerable endoscopic camera device 50 of FIG. 1 , the control wires may be actuated by rotation of control wheels 51, 53. Each control wheel can be rotated to operate a control wire or pair of control wires in a manner exerting push/pull tension on a deflectable distal device portion (not shown, but well-known in the art) to deflect that portion along a first plane, while the other control wheel operates similarly to deflect that portion along a second plane intersecting (e.g., orthogonal to) the first plane. At times, it is desirable to lock that distal device portion into a particular deflected orientation (e.g., so that the operator may execute another task requiring releasing hand contact with one or both control wheels). The illustrated device 50 includes a first brake for the first control wheel 51, with a twistable knob 55 for locking/unlocking an internal brake mechanism that operates along the central rotational axis of the first control wheel 51. The illustrated device 50 includes a second brake for the second control wheel 51, with a lever 57 for locking/unlocking an internal brake mechanism that operates by exerting a braking engagement along the central rotational axis of the second control wheel 53. One or both brake controls 55, 57 require a user to change his/her grip for actuation. Other examples of brake mechanisms are described and illustrated in, for example, U.S. Pat. Nos. 8,864,656; 8,808,168; 8,608,649; 8,641,604; 8,366,604; 6,673,012; and 5,507,717.

It is be desirable to provide braking means that allow a user to "lock" into place the wire-operating elements within the control handle in a manner that will hold the distal end of the steerable catheter in a user-determined deflected or non-deflected orientation. Moreover, there is a need for such a braking means that is operable without a user needing to release other control elements of the steerable catheter, and which may simultaneously brake or even lock more than one control wheel/wire element to as to control (that is reduce or eliminate) deflection of the distal end portion through/along all planes.
JP 2005160790 describes an endoscope having bending mechanisms 41, 42 for bending a bending part of the endoscope. The endoscope also has bending braking mechanisms 83, 93 for applying braking to the bending operation mechanisms 41, 42, and a clicking mechanism 88, 98 associated with the operation of the bending braking mechanisms 83, 93 which can be locked.

### BRIEF SUMMARY

The invention is set out in independent claims 1 and 9.

In one aspect, embodiments disclosed herein may include a steerable catheter with a braking mechanism, where a frictionally-engaging braking element engages actuation/control spools of the steerable catheter in a lockable manner that will inhibit (up to and including preventing) movement of a distal end catheter portion out of a selected deflected conformation. The angle of force and the structures of the present embodiments differ from prior braking mechanisms including that the present embodiments use a brake-actuation axis that is orthogonal to (rather than coaxial or nearly coaxial with) a central rotational axis of control handles and spools of the steerable catheter.

In some embodiments of a steerable catheter with a braking mechanism, the steerable catheter includes a proximal handle body from which extends distally an elongate steerable catheter body with a deflectable distal catheter body end. The proximal handle body includes: at least a first control wheel in mechanical communication, via a first spool, with the distal catheter body end, where the at least a first control wheel and the first spool are rotatable around a common control wheel axis; a brake knob mounted rotatingly to the proximal handle body and configured to rotate around a brake knob axis that is non-coaxial with the control wheel axis; a brake arm in mechanical communication with the brake knob and disposed between the brake knob and the first spool, said brake arm including at least one spool-engagement surface; and ramped and planar handle body surfaces that complementarily interface with ramped and planar brake knob surfaces such that (*i*) in a first brake-actuation knob position the at least one spool-engagement surface does not contactingly engage the first spool, and (*ii*) in a second brake-actuation knob position the at least one spool-engagement surface does contactingly engage the first spool.

In another aspect, embodiments disclosed herein may include a steerable catheter with a brake mechanism that includes a knob mounted to a catheter handle body, said knob being rotatable (relative to the handle body) around a knob axis; where the catheter handle body includes at least one steering control spool rotatable around a spool axis that is orthogonal to the knob axis; a handle ramped surface separated from a handle stop-surface, where a handle-contacting knob surface is movable across the handle ramped surface and is not movable past the handle stop-surface; and a frictional braking element in mechanical communication (along the knob axis) with the knob, said braking element disposed adjacent the at least one spool and actuatable into and out of contact with a contact surface of the at least one spool upon rotation of the knob around the knob axis.

### BRIEF DESCRIPTION OF THE DRAWINGS

FIG. 1 shows a prior steerable catheter device;
FIG. 2A shows a steerable catheter device embodiment handle and distal, deflected tip portion;
FIG. 2B shows a sagittal (vertical plane) section view of FIG. 2A taken along line 2B-2B;
FIG. 2C is a partially disassembled perspective view of the embodiment of FIG. 2A;
FIG. 3A shows the ramped recessed handle body surface that receives a brake knob;
FIG. 3B shows an underside view of the brake knob;
FIG. 3C shows the brake knob cap;
FIG. 3D shows the brake arm with brake shoes; and
FIGS. 4A-4C depict brake knob actuation states of a brake mechanism.

### DETAILED DESCRIPTION

Various embodiments are described below with reference to the drawings in which like elements generally are referred to by like numerals. The relationship and functioning of the various elements of the embodiments may better be understood by reference to the following detailed description. However, embodiments are not limited to those illustrated in the drawings. It should be understood that the drawings are not necessarily to scale, and in certain instances details may have been omitted that are not necessary for an understanding of embodiments disclosed herein, such as - for example -conventional fabrication and assembly.

Generally, embodiments disclosed herein relate to a structure and system for securely attaching the proximal ends of control wires (including any kind of control fiber, regardless of construction material) to the control spool(s) of a steerable catheter. In the most preferred embodiments, the structure and system include means for tuning - that is finely adjusting - relative tension of each of those control wires between the proximal end and a permanently/securely attached distal control wire end attached more distally within the steerable device. Too much or too little tension in each of the control wires (on its own, and more particularly in relation to the other control wire(s)) can cause premature or otherwise undesired deflection of the steerable device and/or may cause the steerable device to operate in a manner that is not desired or predictable. During assembly of a steerable catheter device, the system can be used to take up slackness one or all control wires.

The invention is defined by the claims, may be embodied in many different forms, and should not be construed as limited to the embodiments set forth herein; rather, these embodiments are provided so that this disclosure will be thorough and complete, and will fully convey enabling disclosure to those skilled in the art. As used in this specification and the claims, the singular forms "a," "an," and "the" include plural referents unless the context clearly dictates otherwise.

The terms "proximal" and "distal" are used herein in the common usage sense where they refer respectively to a handle/doctor-end of a device or related object and a tool/patient-end of a device or related object. The terms "about," "generally," "substantially," and other generalizing terms, when used with reference to any volume, dimension, proportion, or other quantitative value is intended to communicate a definite and identifiable value within the standard parameters and variations that would be understood by one of skill in the art, and should be interpreted to include at least any legal equivalents (same or substantially similar function, manner of operation, and result at the element and assembly levels), minor but functionally-insignificant variants, and encompassing a numerical range that includes at least mathematically significant figures (although not required to be as broad as the largest range thereof), as well as including physical embodiments that encompass normal variations in manufacturing tolerances.

The term "control wire" (including just "wire") is used herein to denote the elongate members that connect a control surface of a steerable catheter with a deflectable distal portion of the catheter, and it may include metallic, polymeric, and/or other materials including - by way of nonlimiting example - ultrahigh molecular weight polyethylene (UHMWPE) yarn (e.g., Dyneema^{™}), aramid fibers, monofilament line, multifilament/multifilar cable, and/or other materials that preferably have high tensile strength with low longitudinal stretch so as to provide predictable operation behavior. Metallic control wires can include stainless steel, NiTi, and/or high carbon steel alloy "music wire". Various other polymers may also be used within the scope of the present disclosure. The wire shape may be round (circular or non-circular in transverse section), flat (including rectilinear or with flat surfaces in transverse section). With regard to the physical construction, it may also be braided and/or twisted and optionally may be fused along at least some lengthwise portions. Also, the wires can be coated: for example a low friction polymer coating may be used over a substrate (or as a construction material) in order to reduce dynamic friction within the device's wire lumen(s) so as to provide a more controlled, repeatable response to actuation than higher-friction materials. One preferred material includes UHMWPE braided fiber. With regard to distal attachment of the control wire(s), a multifilar, braided, or other structure is preferred, which may be at least partially frayed or otherwise partially disaggregated (e.g., in order to provide greater surface area than a unitary aggregated wire structure, as described further below).

One example of a control wire may include a 4x-50 Denier ultrahigh tenacity polyethylene braid having a very small outer diameter of about 0.18 mm (measured in accordance with ASTM D-1907); high strength (about 5.6 kg, and at least equal to or greater than 4.75 kg, measured in accordance with ASTM D-6775); low longitudinal stretch/ elongation (about 5%, ±2%, measured in accordance with ASTM D-6775) (e.g., as available from Textile Development Associates Inc. of Brookfield, Conn.). Certain preferred control wire embodiments include or may even consist of high modulus fiber material that is nonconductive and/or substantially nonstretching. In one embodiment, a high modulus fiber control wire material may be braided. One such high modulus fiber material can be a High Molecular Density Polyethylene, a melt spun liquid crystal polymer fiber rope, or a spun para-aramid fiber polymer, or a high strength ceramic fiber. In some embodiments, a high modulus fiber control wire material may have a tensile strength in a range of about 300 ksi (2,000 MPa) to 1,500 ksi (10,400 MPa), and/or a tensile modulus in the range of about 5,000 ksi (35,000 MPa) to about 20,000 ksi (140,000 MPa).

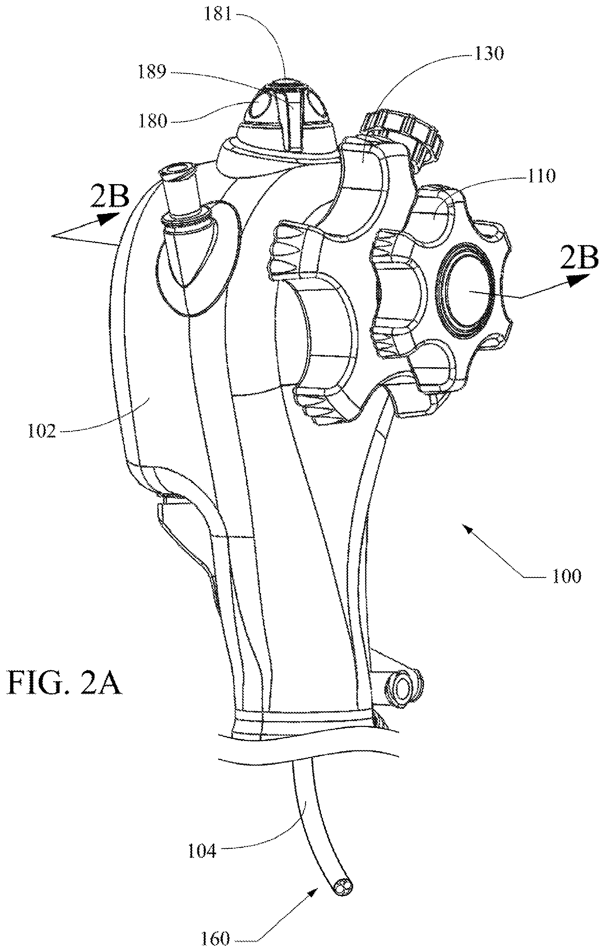

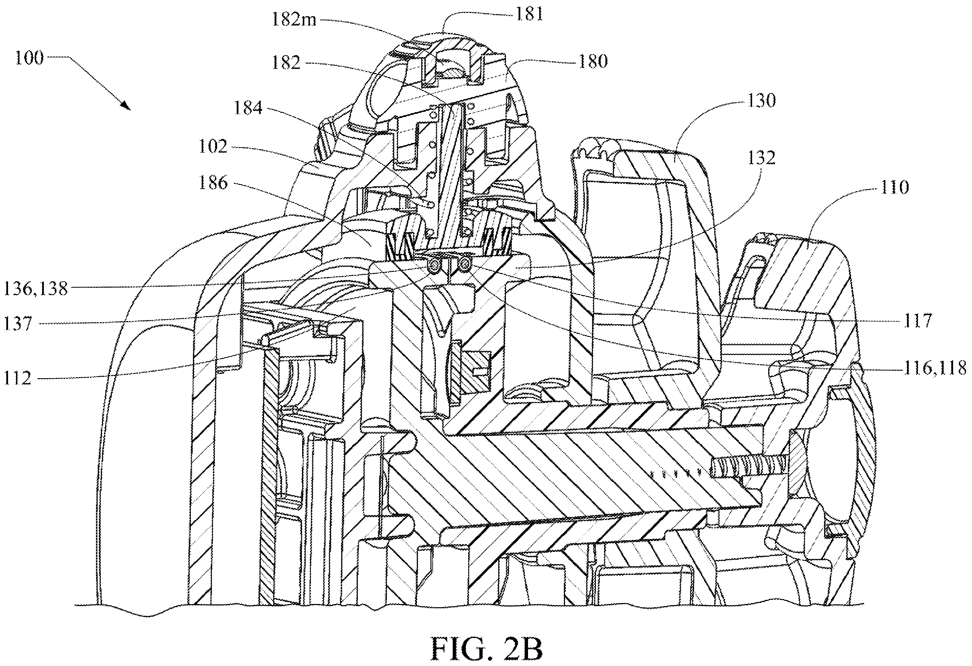

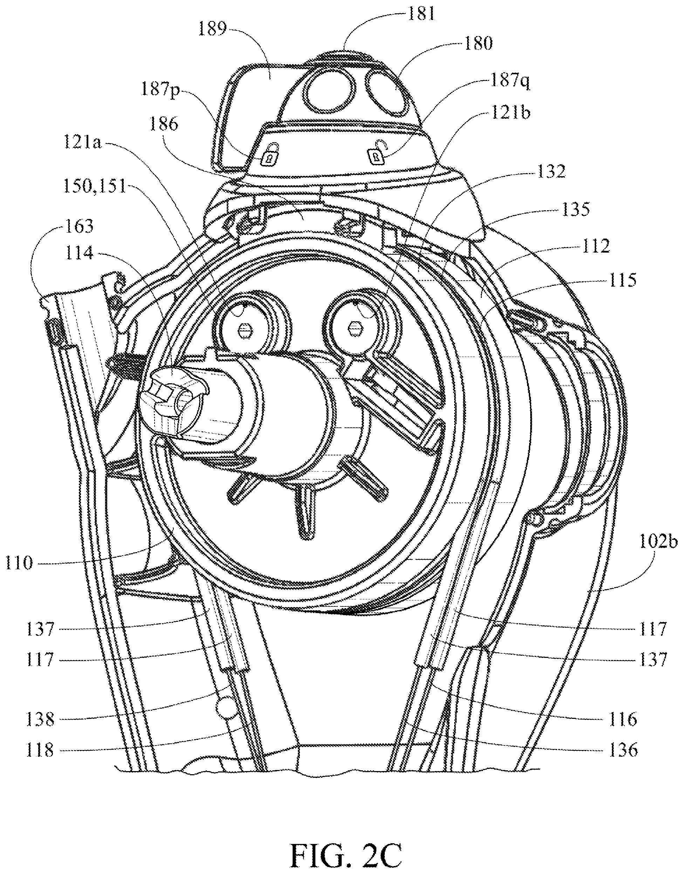

One embodiment of a steerable catheter device 100 is described with reference to FIG. 2. The steerable catheter device 100 includes a proximal control handle body 102 with a steerable catheter body 104 extending distally therefrom (which may have a default straight linear configuration, and for which is illustrated only a deflected distal end terminal lengthwise portion). Various embodiments may include one or more different steering control means known in the art. This illustrated embodiment includes a pair of control wheels, with an outer control wheel 110 and an inner control wheel 130. As set forth in greater detail below (including with reference to FIGS. 2B-2C), the outer control wheel 110 is disposed in mechanical communication with a pair of control wires that are operable, upon wheel rotation, to deflect at least the end portion 160 of the catheter body 104 along a first plane, and the inner control wheel 130 is disposed in mechanical communication with another pair of control wires that are operable, upon wheel rotation, to deflect the catheter body 104 along a second plane that may be generally orthogonal to the first plane, and is at least somewhat offset from that first plane. Simultaneous or sequential operation of the outer and inner wheels 110, 130 preferably can deflect the distal end portion 160 of the catheter body 104 in any direction around a 360-degree circle defined generally by a circumference of the catheter.

Steering mechanisms using control wires are well-known in the art including in U.S. Pat. Pub. No. 2015/0366435 to Williams.

The overall control structure described is also well known in the steerable device art, including particularly the endoscope art, but those devices lack the currently disclosed finely-controlled mechanism for efficient and effective tensioning of control wires. Certain embodiments in keeping with the present disclosure may include at least one visualization element (as well as supporting hardware and/or software, not shown - but well-known in the art and readily understandable as using electrical and/or optical devices such as CCD, fiber optic, CMOS, etc.) for use of such embodiments as endoscopic devices including, for example, as a cholangioscope configured for use with and through a larger endoscope.

A sagittal (vertical plane) section view of FIG. 2A taken along line 2B-2B is shown in FIG. 2B to illustrate the physical arrangement of components, from which those of skill in the art will understand the structures and functions described herein. A partially disassembled view of the control handle portion of the steerable catheter device 100 is shown in FIG. 2C, where a portion of the body 102, outer control wheel 110, and the inner control wheel 130 are removed, and the spool assemblies therein are shown in more detail. The outer control wheel 110 engages a shaft 114 of, and controls rotation of, an outer spool 112 around a common central rotational axis (that preferably is orthogonal to the generally circular handle and spool). The outer spool 112 includes a circumferential groove 115 around its outer circumferential surface, which groove 115 receives a tube 117 through which extend the proximal end regions of opposed first and second control fibers 116, 118. The outer spool 112 includes two gear-mounting apertures 121a, 121b, each of which receives and forms a rotation-permitting engagement with the split mounting end 152 of a gear 150. Each spool includes at least one face surface intersecting the spool axis (preferably having the major face congruent with a plane that is orthogonal to that spool rotational axis) and at least one circumferential surface that includes or is included by a contact surface that may frictionally be contacted by a brake mechanism.

The inner control wheel 130 engages a shaft 134 of, and controls rotation of, an inner spool 132. The inner spool 132 includes a circumferential groove 135 around its outer circumferential surface, which groove 135 receives a tube 137 through which extend the proximal end regions of opposed third and fourth control fibers 136, 138. The proximal end terminus of each control wire (not shown) is secured to its respective spool. Those of skill in the art will appreciate that rotary actuation of the outer control wheel 110 effects corresponding rotary actuation of the outer spool 112, while rotary actuation of the inner control wheel 130 effects corresponding rotary actuation of the outer spool 132, and that respective distal attachments of each control fiber to/in the distal end lengthwise portion 160 of the catheter body 104 will provide for controllable deflection.

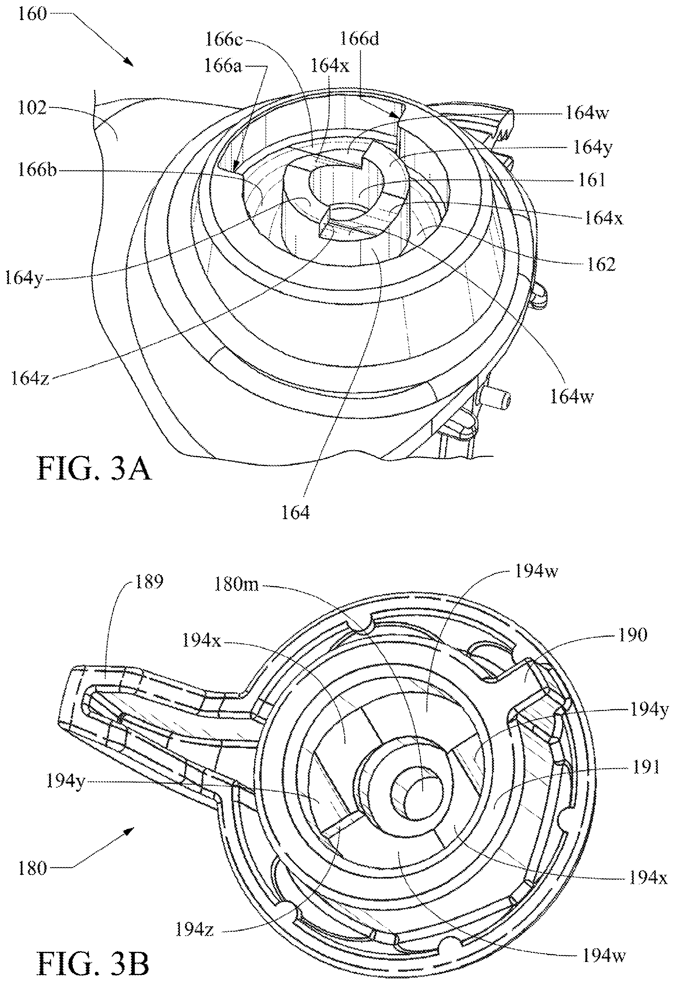

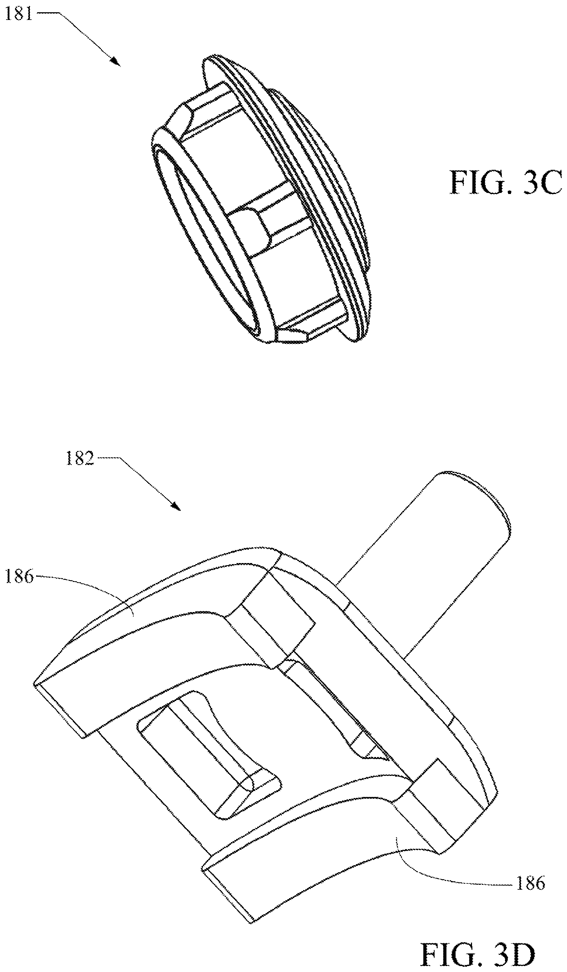

As shown in FIGS. 2B and 2C, the outer spool shaft 114 extends through and beyond a central passage of the inner spool 132 and its shaft 134. A brake assembly is also depicted, including a brake-actuation knob 180 with cap 181, brake arm 182 including brake shoes 186 (which may be structurally and compositionally integral with or different than the rest of the arm),and a biasing element (illustrated here as a coil spring 184), elements of which are shown in FIGS. 3B-3D. In both FIGS. 2B and 2C, the illustrated brake assembly is shown as having two brake shoes 186 that serve as spool-engaging surfaces, where each one is shown in engaging contact with a respective one of the spools 112, 132. The biasing element 184 biases the brake arm 182 away from an inward-facing surface of the housing 102 toward/into this engaging contact, which preferably provides sufficient force to resist (up to and including to prevent) rotation of the spools relative to the brake arm 182 when fully engaged. More particularly, the spring (or other biasing element known or developed in the art) is selected and/or configured to exert a predetermined force when assembled. The contact shown is between the brake shoes 186 and an outer circumferential surface of the respective spools 112, 132. The axial direction of the biasing contact force preferably is at least substantially orthogonal to the central rotational axis of the spools 112, 132 and control wheels 110, 130, and is not coaxial with that central rotational axis (being at least, or greater than, about 30° off that central rotational axis).

In other embodiments, a single brake shoe, or an integrated same-material portion of the brake arm 182 may serve as the spool-engaging surface(s). In other embodiments, more or fewer elements may be used to contact and inhibit (up to and including preventing) movement of the spools by providing a predetermined force of the brake arm toward the spool - and preferably toward, and orthogonal with, the common rotational axis of the spool(s) and control wheel(s). This structure and functionality provides an operator of a steerable catheter to deflect the distal end portion to a desired conformation, then actuate the brake mechanism to inhibit spool movement and thereby hold that conformation while the operator frees up his/her hands to perform other tasks.

In the illustrated embodiment, a rotating knob 180 interfaces with the housing body 102 of the steerable catheter in order to actuate (that is, to engage and/or disengage) the brake mechanism relative to the spools. In this embodiment, the central rotational axis of the knob 180 is coaxial with the engagement/disengagement axis of the brake arm 182 and is orthogonal relative to the central rotational axis of the spools 112, 132 and control wheels 110, 130. The brake mechanism is configured so that rotation of the knob 180 in a first direction advances the brake arm toward the spools, and rotation in a second/opposite direction retracts the brake arm away from the spools. In the illustrated embodiment, the coil spring 184 provides biasing force against the brake arm 182 toward the spools. The knob 180 is attached to the brake arm 182 in a manner allowing the knob to rotate around the brake arm, but providing for fixed attachment and connected movement of the brake arm together with the knob along the longitudinal axis of the brake arm away from the spools. This attachment may be effected by a threaded connector (182m in FIG. 2B, disposed through a knob aperture 180m) or other means that preferably allow for adjustment/tuning during assembly (in coordination with the biasing means 184, which may be a coil spring, leaf spring, or any other appropriate biasing means).

This operation for engagement/disengagement of the brake arm 182 with the spools is effected by a complementary interface between the underside of the knob 180 (shown in FIG. 3B) and a recessed portion of the handle body housing 102 (shown in FIG. 3A). As will be appreciated by those of skill in the art, these complementary surfaces are contoured and dimensioned to provide a path of motion that will selectably direct the brake arm into contact with, and retract the brake arm from contact with, the spools. Changes in the contours and/or dimensions may be used to increase or decrease the amount of force directed into full-contact engagement between the brake arm and the spools. In the illustrated embodiment, the contact provided by the complementary interface (described in more detail below) may be characterized as light contact, which is enhanced by the bias of the biasing element 184.

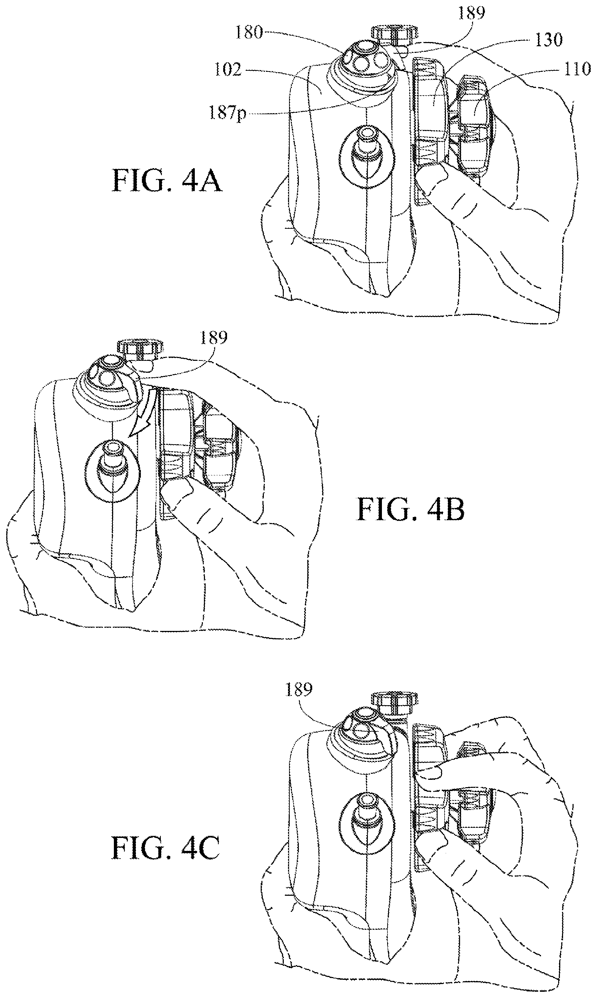

The exact quantitative measurements of the frictional and other forces will vary depending upon the materials of the brake arm (including brake shoes, if present), the spools, the control wires, and the catheter body. However, those of skill in the art will be able to provide and fine-tune this mechanism with reference to the present disclosure. At the very least, locked-in engagement of the brake mechanism will hold the spools in place when they are oriented in a manner that deflects the catheter distal end portion 160, resisting any implicit/natural tendency of the catheter or other elements to straighten the catheter body or otherwise move. In certain preferred embodiments, an operator may still rotate one or both control wheels 110, 130 in a manner moving one or both spools - even while engaged with the brake - without damaging any components, but such an operation will require exerting greater force than operating the wheels and spools without the brake engaged. For example, this may allow a user to deflect the catheter, lock in the brake (e.g., as shown in FIGS. 4A-4C), direct a tool through a working channel of the catheter, then -with the brake engaged- move (e.g., "fine tune") the location of the deflected catheter end to a desired location. However, in preferred embodiments the locked-in deflected end will not move unless the operator actuates one or both control wheels with sufficient force to overcome the frictional engagement of the brake mechanism with the spool(s).

The brake mechanism's operation, including by complementary interaction of ramped surfaces, may be understood with reference to FIGS. 2B, 3A, 3B, 3D, and 4A-4C. The brake arm 182 shown in FIG. 3D includes a post 182a, a foot 182b, and the brake shoes 186, where the illustrated embodiment shows the foot and shoes as being curved to conform in a matching complementary manner with the outer circumferential surface of the spools so as to fully frictionally engage therewith. The brake arm post 182a passes through a central opening 161 of the knob-receiving recess 160 and is received rotatably into the knob 180 so that movement of the knob along the post's longitudinal central axis will move the arm reciprocally along that axis (which is also the central rotational axis of the knob).

The recess 160 of the housing 102 includes an outer track, a channel 162, and an inner annulus 164, which allow transit interface therewith, respectively, of a camming tab 190, a ring 191, and an inner track within the ring. When the knob 180 is engaged into the recess 160, the camming tab 190 rides along a ramped surface 166c and planar surface 166b of the outer track between end termini 166a, 166d. At the same time ramped and planar surfaces of the inner annulus 164 will complementarily engage ramped and planar surfaces of the knob's inner track. The inner annulus 164 includes radially-opposite lower planar surfaces 164w that are separated from higher planar surfaces 164y by intervening ramped surfaces 164x. This structure provides a stop-surface 164z that are shown as substantially or exactly perpendicular to the lower planar surfaces 164w. On the ring 191, the inner track includes radially-opposite lower planar surfaces 194w that are separated from higher planar surfaces 194y by intervening ramped surfaces 194x. This structure provides a stop-surface 194z that are shown as substantially or exactly perpendicular to the lower planar surfaces 194w.

In a first, unlocked/unbraked, state, with the knob 180 in the position shown in FIG. 4A, the brake arm (including its brake shoe(s), if present) is not contacting the spools, so that the operator can freely rotate the control wheels and spools to deflect the catheter. An advantage of the present embodiments is also shown in FIGS. 4A-4B, where the operator can use a single finger to actuate/rotate the knob 180 without releasing or changing grip on the control wheels 110, 130. Stated differently, the knob is located and oriented to be reachable by a single digit of a user's hand, other digits of which are simultaneously operably grasping one or both control wheel(s). Internally, in the unlocked/ unbraked state, the camming tab 190 contacts both the housing's planar surface 166b and the unbraked terminal end 166a of the housing's outer track, where - in this position - the knob holds the attached brake arm away from the spools (where the knob's cap 181 is at its greatest distance from the housing 102). This unbraked state is aided or at least complemented by the interaction of the opposed, but complementary engagement of ramped and planar surfaces of the knob's inner track with ramped and planar surfaces of the housing's inner annulus. Specifically, the knob's higher planar surfaces 194y contact the inner annulus higher surfaces 164y. An externally visible portion of the housing may include visual indicia (e.g., showing an image and/or wording for unbraked/unlocked) corresponding to the position of the exterior knob tab 189 in this unbraked state.

In a second, locked/braked, state, with the knob 180 in the position shown in FIG. 4C, the brake arm (including its brake shoe(s), if present) is contacting the spools in the manner shown in FIGS. 2B-2C, so that the control wheels and spools are held in place and a deflected position of the catheter is retained without the operator having to hold one or both control wheels in place. Internally, in the locked/braked state, the camming tab 190 contacts both the housing's ramped surface 166c and the braked terminal end 166d of the housing's outer track. In this position, the knob 180 moves the attached brake arm 182 toward the spools (so that the knob's cap 181 is at its nearest distance from the housing 102, and the biasing means 184 operates to press/bias the brake arm toward/against the spool(s)). This braked state is aided or at least complemented by the interaction of the opposed, but complementary engagement of ramped and planar surfaces of the knob's inner track with ramped and planar surfaces of the housing's inner annulus. Specifically, the knob's higher planar surfaces 194y contact the inner annulus lower planar surfaces 164w. An externally visible portion of the housing may include visual indicia (e.g., showing an image and/or wording for braked/locked) corresponding to the position of the exterior knob tab 189 in this braked state, where FIG. 4A shows - by way of nonlimiting example - an iconographic image of a closed lock that is aligned with the outer knob tab 189 in FIG. 4C, and the locked/unlocked icons 187p/187q in FIG. 2C.

The combination of contact between the knob 180 and housing recess 160 with the biasing means 184 may also provide, in certain embodiment, tactile and/or auditory feedback for braking/locking engagement and/or for unbraking/unlocking disengagement. For example, as an operator actuates the knob 180 by rotation from the position shown in FIG. 4A to the position shown in FIG. 4C, the operator may feel and/or hear a "click" as the tab 190 moves into seated contact against the braked terminal end 166d of the housing's outer track. Similarly, when an operator disengages the brake mechanism by rotating the knob 180 from the position shown in FIG. 4C to the position shown in FIG. 4A, the operator may feel and/or hear a "click" as the tab 190 moves into seated contact against the unbraked terminal end 166a of the housing's outer track. One or more of the visual, tactile, and auditory indicia will provide an operator with increased confidence and security regarding the operative state of the distal catheter end portion 160 (e.g., whether it is freely moving/deflectable, or is substantially locked into a particular conformation). As noted above, being substantially locked into place by frictional contact of the brake arm with the spool(s) may still -in some embodiments- permit an operator to exert extra force and still move the control wheels in a limited manner that may be used to fine-tune position/conformation of the distal catheter end portion 160.

With regard to particular construction materials those of skill in the art, when informed by the present disclosure, will appreciate that many variant options are possible for exact dimensions and construction materials of the brake arm, biasing means, brake shoe(s)(if present), spool outer circumferential surfaces, etc. An effective brake mechanism assembly according to the presently disclosed inventive embodiments will provide a combination of normal force and dynamic friction that offer the braking functionality described herein. In one embodiment, the brake arm 182 may be constructed of polycarbonate, and the brake shoe(s) 186 may be constructed of an elastic high-friction moldable polymer (e.g., Versaflex^{™} OM 1040X, available from PolyOne of Avon Lake, Ohio). The spools contacted by the brake shoe(s) may be made of HOPE (highdensity polyethylene) or nylon (e.g., Hylon^{™} 1000N), which will provide good braking contact as well as provide the mechanical strength and other properties needed for fine control of the control wires. The surface of the spool(s) and/or brake shoe(s) may also be textured to enhance frictional contact.

Those of skill in the art will appreciate that features described herein for different embodiments may be combined with each other and/or with currently-known or future-developed technologies within the scope of the claims. References herein to any industry standards (e.g., ASTM standards, and product identifiers such as particular polymers, as well as any trademarks) are defined as complying with the currently published standards and corresponding quantitatively and qualitatively defined specifications as of the original filing date of this disclosure unless expressly otherwise defined herein. And, it should be understood that the following claims, interpreted in accordance with The Protocol on Interpretation of Article 69 EPC, define the scope of this invention.

## Claims

1. A steerable catheter (100) with braking mechanism, the steerable catheter comprising:
a proximal handle body (102) from which extends distally an elongate steerable catheter body (104) including a deflectable distal catheter body end;
where the proximal handle body includes:
at least a first control wheel (110) in mechanical communication, via a first spool (112), with the distal catheter body end, where the at least a first control wheel and the first spool are rotatable around a common control wheel axis;
a brake knob (180) mounted rotatingly to the proximal handle body and configured to rotate around a brake knob axis that is orthogonal to the control wheel axis;
a brake arm (182) in mechanical communication with the brake knob and disposed between the brake knob and the first spool, said brake arm including at least one spool-engagement surface (186); and
ramped and planar handle body surfaces (164w-y, 166b-c) that complementarily interface with ramped and planar brake knob surfaces (194w-y) such that
in a first brake-actuation knob position the at least one spool-engagement surface does not contactingly engage the first spool, and
in a second brake-actuation knob position the at least one spool-engagement surface does contactingly engage the first spool.

2. The steerable catheter of claim 1, where the at least one spool-engagement surface is biased away from the knob and toward the control wheel axis.

3. The steerable catheter of claim 1 or 2, where the knob includes a stop tab (190), which - when the knob is disposed in the first brake-actuation knob position - contacts a first stop-surface (166a) of the handle body, and which - when the knob is disposed in the second brake-actuation knob position - contacts a second stop-surface (166d) of the handle body.

4. The steerable catheter of claim 3, where the knob's stop tab contacts at least one of the ramped and planar handle body surfaces (166b-c) between the first stop-surface and the second stop-surface.

5. The steerable catheter of any of the preceding claims, where the knob is confined to a mechanically-limited arc of rotation, one end of which arc corresponds to the knob being in the first brake-actuation knob position and the stop tab contacting a first handle stop-surface, and an opposite end of which arc corresponds to the knob being in the second brake-actuation knob position and the stop tab contacting a second handle stop-surface; optionally where the at least one spool-engagement surface is configured as at least one brake shoe.

6. The steerable catheter of any of the preceding claims, where the knob is located in a position reachable by a digit of a user's hand, other digits of which are simultaneously operably grasping the at least a first control wheel.

7. The steerable catheter of any of the preceding claims, constructed such that when the knob is in the second brake-actuation knob position, the spool-engagement surface contacting engagement with the at least a first spool provides sufficient friction to inhibit said spool from rotating freely, and thereby corresponds to inhibiting change in a deflected state of the distal catheter body end.

8. The steerable catheter of any of the preceding claims, where a coil spring (184) biases the at least one spool-engagement surface away from the knob and toward the control wheel axis with a predetermined amount of force such that the spool-engagement surface contacting engagement with the at least a first spool provides sufficient friction to inhibit said spool from rotating freely and inhibits change in a deflected state of the distal catheter body end.

9. A steerable catheter with brake mechanism, the brake mechanism comprising:
a knob mounted to a catheter handle body, said knob being rotatable, relative to the handle body, around a knob axis;
where the catheter handle body includes at least one steering control spool rotatable around a spool axis that is orthogonal to the knob axis;
a handle ramped surface separated from a handle stop-surface, where a handle-contacting knob surface is movable across the handle ramped surface and not movable past the handle stop-surface; and
a frictional braking element in mechanical communication, along the knob axis, with the knob, said braking element disposed adjacent the at least one spool and actuatable into and out of contact with a contact surface of the at least one spool upon rotation of the knob around the knob axis.

10. The steerable catheter of claim 9, where the braking element is biased away from the knob and toward the at least one spool.

11. The steerable catheter of claim 9 or 10, where the handle-contacting knob surface includes a knob ramped surface and includes a knob stop-surface substantially perpendicular to a knob ramped surface.

12. The steerable catheter of claim 11, where a first actuation state corresponds to the knob stop-surface contacting the handle stop-surface and the frictional braking element contacting the at least one spool with sufficient force to inhibit spool rotation.

13. The steerable catheter of claim 11 or 12, where the braking element is biased away from the knob and toward the spool; optionally where the knob has a mechanically-limited arc of rotation, one end of which arc is defined by the knob stop-surface contacting the handle stop-surface, and an opposite end corresponding with the braking element not inhibiting rotation of the at least one spool.

14. The steerable catheter of claim 12 or claim 13, where the knob is located in a position reachable and rotatable by a digit of a user's hand, other digits of which hand are simultaneously operably contacting at least a first control wheel, which control wheel shares a common rotational axis with the at least one spool.

15. The steerable catheter of any of claims 12 to 14, where a braking element portion, actuatable into and out of contact with a contact surface of the at least one spool, comprises a brake shoe that biased away from the knob and toward the at least one spool by a coil spring disposed between the brake shoe and the catheter handle body.

## Patentansprüche

1. Lenkbarer Katheter (100) mit Bremsmechanismus, wobei der lenkbare Katheter Folgendes umfasst:
einen proximalen Griffkörper (102), von dem sich distal ein länglicher lenkbarer Katheterkörper (104) erstreckt, der ein auslenkbares distales Katheterkörperende aufweist,
wobei der proximale Griffkörper Folgendes aufweist:
mindestens ein erstes Steuerrad (110) in mechanischer Kommunikation über eine erste Spule (112) mit dem distalen Katheterkörperende, wobei das mindestens eine erste Steuerrad und die erste Spule um eine gemeinsame Steuerradachse drehbar sind,
einen Bremsknopf (180), der drehbar an dem proximalen Griffkörper montiert und dazu ausgestaltet ist, sich um eine Bremsknopfachse zu drehen, die orthogonal zu der Steuerradachse verläuft,
einen Bremsarm (182), der in mechanischer Verbindung mit dem Bremsknopf steht und zwischen dem Bremsknopf und der ersten Spule angeordnet ist, wobei der Bremsarm mindestens eine Spuleneingriffsfläche (186) aufweist, und
rampenförmige und planare Griffkörperflächen (164w-y, 166b-c), die komplementär mit rampenförmigen und planaren Bremsknopfflächen (194w-y) koppeln, so dass
die mindestens eine Spulenangriffsfläche die erste Spule in einer ersten Bremsbetätigungsknopfposition nicht berührend in Eingriff nimmt, und
die mindestens eine Spulenangriffsfläche die erste Spule in einer zweiten Bremsbetätigungsknopfposition berührend in Eingriff nimmt.

2. Lenkbarer Katheter nach Anspruch 1, wobei die mindestens eine Spuleneingriffsfläche von dem Knopf weg und zu der Steuerradachse hin vorgespannt ist.

3. Lenkbarer Katheter nach Anspruch 1 oder 2, wobei der Knopf eine Anschlagnase (190) aufweist, die - wenn der Knopf in der ersten Bremsbetätigungsknopfposition angeordnet ist - eine erste Anschlagfläche (166a) des Griffkörpers berührt und die - wenn der Knopf in der zweiten Bremsbetätigungsknopfposition angeordnet ist - eine zweite Anschlagfläche (166d) des Griffkörpers berührt.

4. Lenkbarer Katheter nach Anspruch 3, wobei die Anschlagnase des Knopfs die rampenförmige und/oder die planare Griffkörperoberfläche (166b-c) zwischen der ersten Anschlagfläche und der zweiten Anschlagfläche berührt.

5. Lenkbarer Katheter nach einem der vorhergehenden Ansprüche, wobei der Knopf auf einen mechanisch begrenzten Drehbogen beschränkt ist, dessen eines Ende der Anwesenheit des Knopfs in der ersten Bremsbetätigungsknopfposition und dem Berühren einer ersten Griffanschlagfläche durch die Anschlagnase entspricht, und dessen entgegengesetztes Ende der Anwesenheit des Knopfs in der zweiten Bremsbetätigungsknopfposition und dem Berühren einer zweiten Griffanschlagfläche durch die Anschlagnase entspricht, optional wobei die mindestens eine Spuleneingriffsfläche als mindestens eine Bremsbacke ausgestaltet ist.

6. Lenkbarer Katheter nach einem der vorhergehenden Ansprüche, wobei sich der Knopf in einer Position befindet, die durch einen Finger der Hand eines Benutzers erreichbar ist, wobei andere Finger gleichzeitig das mindestens eine erste Steuerrad bedienungsmäßig ergreifen.

7. Lenkbarer Katheter nach einem der vorhergehenden Ansprüche, der so konstruiert ist, dass die berührende Ineingriffnahme der mindestens einen ersten Spule durch die Spuleneingriffsfläche, wenn sich der Knopf in der zweiten Bremsbetätigungsknopfposition befindet, für ausreichende Reibung sorgt, um die Spule an einem freien Drehen zu hindern, und dadurch dem Hemmen einer Änderung in einem ausgelenkten Zustand des distalen Katheterkörperendes entspricht.

8. Lenkbarer Katheter nach einem der vorhergehenden Ansprüche, wobei eine Schraubenfeder (184) die mindestens eine Spuleneingriffsfläche mit einem vorbestimmten Kraftbetrag von dem Knopf weg und zu der Steuerradachse hin vorspannt, so dass die berührende Ineingriffnahme der mindestens einen ersten Spule durch die Spuleneingriffsfläche für ausreichende Reibung sorgt, um zu verhindern, dass sich die Spule frei dreht, und eine Änderung in einem ausgelenkten Zustand des distalen Katheterkörperendes verhindert.

9. Lenkbarer Katheter mit Bremsmechanismus, wobei der Bremsmechanismus Folgendes umfasst:
einen Knopf, der an einem Kathetergriffkörper montiert ist, wobei der Knopf bezüglich des Griffkörpers um eine Knopfachse drehbar ist,
wobei der Kathetergriffkörper mindestens eine Lenksteuerspule aufweist, die um eine Spulenachse drehbar ist, die orthogonal zu der Knopfachse verläuft,
eine Grifframpenfläche, die von einer Griffanschlagfläche getrennt ist, wobei eine Griffkontaktknopffläche über die Grifframpenfläche bewegbar, aber nicht an der Griffanschlagfläche vorbei bewegbar ist, und
ein Reibbremselement in mechanischer Kommunikation entlang der Knopfachse mit dem Knopf, wobei das Bremselement benachbart zu der mindestens einen Spule angeordnet ist und bei Drehung des Knopfs um die Knopfachse in und außer Kontakt mit einer Kontaktfläche der mindestens einen Spule betätigbar ist.

10. Lenkbarer Katheter nach Anspruch 9, wobei das Bremselement von dem Knopf weg und zu der mindestens einen Spule hin vorgespannt ist.

11. Lenkbarer Katheter nach Anspruch 9 oder 10, wobei die Griffkontaktknopffläche eine Knopframpenfläche aufweist und eine Knopfanschlagfläche aufweist, die im Wesentlichen senkrecht zu einer Knopframpenfläche verläuft.

12. Lenkbarer Katheter nach Anspruch 11, wobei ein erster Betätigungszustand dem Berühren der Griffanschlagfläche durch die Knopfanschlagfläche und dem Berühren der mindestens einen Spule durch das Reibbremselement mit ausreichender Kraft entspricht, um die Spulendrehung zu hemmen.

13. Lenkbarer Katheter nach Anspruch 11 oder 12, wobei das Bremselement von dem Knopf weg und zu der Spule hin vorgespannt ist, optional, wenn der Knopf einen mechanisch begrenzten Drehbogen aufweist, dessen eines Ende durch das Berühren der Griffanschlagfläche durch die Knopfanschlagfläche definiert ist, und ein gegenüberliegendes Ende, dem entspricht, dass das Bremselement die Drehung der mindestens einen Spule nicht hemmt.

14. Lenkbarer Katheter nach Anspruch 12 oder Anspruch 13, wobei sich der Knopf in einer Position befindet, die durch einen Finger der Hand eines Benutzers erreichbar und drehbar ist, wobei andere Finger der Hand gleichzeitig mit mindestens einem ersten Steuerrad in Wirkkontakt stehen, wobei das Steuerrad und die mindestens eine Spule eine gemeinsame Drehachse haben.

15. Lenkbarer Katheter nach einem der Ansprüche 12 bis 14, wobei ein Bremselementabschnitt, der in und außer Kontakt mit einer Kontaktfläche der mindestens einen Spule betätigbar ist, eine Bremsbacke umfasst, die durch eine zwischen der Bremsbacke und dem Kathetergriffkörper angeordnete Schraubenfeder von dem Knopf weg und zu der mindestens einen Spule hin vorgespannt ist.

## Revendications

1. Cathéter orientable (100) avec mécanisme de freinage, le cathéter orientable comprenant :
un corps de poignée proximal (102) à partir duquel s'étend distalement un corps de cathéter orientable allongé (104) comprenant une extrémité de corps de cathéter distale déformable ;
le corps de poignée proximal comprenant :
au moins une première roue de commande (110) en communication mécanique, par l'intermédiaire d'une première bobine (112), avec l'extrémité de corps de cathéter distale, l'au moins une première roue de commande et la première bobine étant rotatives autour d'un axe de roue de commande commun ;
un bouton de frein (180) monté en rotation sur le corps de poignée proximal et configuré pour tourner autour d'un axe de bouton de frein qui est orthogonal à l'axe de roue de commande ;
un bras de frein (182) en communication mécanique avec le bouton de frein et disposé entre le bouton de frein et la première bobine, ledit bras de frein comprenant au moins une surface de mise en prise de bobine (186) ; et
des surfaces de corps de poignée en rampe et planes (164w-y, 166b-c) qui s'interfacent de manière complémentaire avec des surfaces de bouton de frein en rampe et planes (194w-y) de manière à ce que
dans une première position de bouton d'actionnement de frein, l'au moins une surface de mise en prise de bobine n'entre pas en contact avec la première bobine, et
dans une seconde position de bouton d'actionnement de frein, l'au moins une surface de mise en prise de bobine entre en contact avec la première bobine.

2. Cathéter orientable selon la revendication 1, l'au moins une surface de mise en prise de bobine étant sollicitée à l'écart du bouton et vers l'axe de roue de commande.

3. Cathéter orientable selon la revendication 1 ou 2, le bouton comprenant une languette d'arrêt (190) qui - lorsque le bouton est disposé dans la première position de bouton d'actionnement de frein - entre en contact avec une première surface d'arrêt (166a) du corps de poignée, et qui - lorsque le bouton est disposé dans la seconde position de bouton d'actionnement de frein - entre en contact avec une seconde surface d'arrêt (166d) du corps de poignée.

4. Cathéter orientable selon la revendication 3, la languette d'arrêt du bouton entrant en contact avec au moins une des surfaces de corps de poignée en rampe et planes (166b-c) entre la première surface d'arrêt et la seconde surface d'arrêt.

5. Cathéter orientable selon l'une quelconque des revendications précédentes, le bouton étant confiné à un arc de rotation mécaniquement limité, dont une extrémité correspond au fait que le bouton est dans la première position de bouton d'actionnement de frein et que la languette d'arrêt est en contact avec une première surface d'arrêt de poignée, et dont une extrémité opposée correspond au fait que le bouton est dans la seconde position de bouton d'actionnement de frein et que la languette d'arrêt est en contact avec une seconde surface d'arrêt de poignée ; éventuellement, l'au moins une surface de mise en prise de bobine étant configurée comme au moins un sabot de frein.

6. Cathéter orientable selon l'une quelconque des revendications précédentes, le bouton étant situé dans une position accessible par un doigt de la main de l'utilisateur, dont d'autres doigts saisissent simultanément de manière opérationnelle l'au moins une première roue de commande.

7. Cathéter orientable selon l'une quelconque des revendications précédentes, construit de telle sorte que lorsque le bouton est dans la seconde position de bouton d'actionnement de frein, la surface de mise en prise de bobine en contact avec l'au moins une première bobine fournit une friction suffisante pour empêcher ladite bobine de tourner librement, et correspond ainsi à l'inhibition du changement d'un état défléchi de l'extrémité de corps de cathéter distale.

8. Cathéter orientable selon l'une quelconque des revendications précédentes, un ressort hélicoïdal (184) sollicitant l'au moins une surface de mise en prise de bobine à l'écart du bouton et vers l'axe de roue de commande avec une quantité de force prédéterminée de sorte que la surface de mise en prise de bobine en contact avec l'au moins une première bobine fournit un frottement suffisant pour empêcher ladite bobine de tourner librement et empêche le changement d'un état défléchi de l'extrémité de corps de cathéter distale.

9. Cathéter orientable avec mécanisme de freinage, le mécanisme de freinage comprenant :
un bouton monté sur un corps de poignée de cathéter, ledit bouton étant rotatif, par rapport au corps de poignée, autour d'un axe de bouton ;
le corps de poignée de cathéter comprenant au moins une bobine de commande de direction rotative autour d'un axe de bobine qui est orthogonal à l'axe de bouton ;
une surface en rampe de poignée séparée d'une surface d'arrêt de poignée, une surface de bouton en contact avec la poignée étant mobile sur la surface en rampe de poignée et ne pouvant pas être déplacée au-delà de la surface d'arrêt de poignée ; et
un élément de freinage par friction en communication mécanique, le long de l'axe de bouton, avec le bouton, ledit élément de freinage étant disposé à côté de l'au moins une bobine et pouvant être mis en contact ou hors contact avec une surface de contact de l'au moins une bobine lors de la rotation du bouton autour de l'axe de bouton.

10. Cathéter orientable selon la revendication 9, l'élément de freinage étant sollicité à l'écart du bouton et vers l'au moins une bobine.

11. Cathéter orientable selon la revendication 9 ou 10, la surface de bouton en contact avec la poignée comprenant une surface en rampe du bouton et comprenant une surface d'arrêt de bouton sensiblement perpendiculaire à une surface en rampe du bouton.

12. Cathéter orientable selon la revendication 11, un premier état d'actionnement correspondant à la surface d'arrêt de bouton en contact avec la surface d'arrêt de poignée et à l'élément de freinage par friction en contact avec l'au moins une bobine avec une force suffisante pour inhiber la rotation de la bobine.

13. Cathéter orientable selon la revendication 11 ou 12, l'élément de freinage étant sollicité à l'écart du bouton et vers la bobine ; éventuellement, le bouton ayant un arc de rotation mécaniquement limité, une extrémité de cet arc étant définie par la surface d'arrêt de bouton en contact avec la surface d'arrêt de poignée, et une extrémité opposée correspondant à l'élément de freinage n'empêchant pas la rotation de l'au moins une bobine.

14. Cathéter orientable selon la revendication 12 ou selon la revendication 13, le bouton étant situé dans une position accessible et pouvant être tournée par un doigt de la main de l'utilisateur, d'autres doigts de cette main étant simultanément en contact opérationnel avec au moins une première roue de commande, laquelle roue de commande partage un axe de rotation commun avec l'au moins une bobine.

15. Cathéter orientable selon l'une quelconque des revendications 12 à 14, une partie d'élément de freinage, actionnable en contact et hors de contact avec une surface de contact de l'au moins une bobine, comprenant un sabot de frein qui est sollicité à l'écart du bouton et vers l'au moins une bobine grâce à un ressort hélicoïdal disposé entre le sabot de frein et le corps de poignée de cathéter.
